# EUROPEAN PATENT SPECIFICATION

(11) **EP 1 866 033 B1**
(45) Date of publication and mention of the grant of the patent: **19.04.2017**
(21) Application number: 06721287.8
(22) Date of filing: 24.03.2006
(51) Int. Cl.: A61K 8/06, A61Q 17/04, A61K 8/04

(54) **SUNSCREEN AEROSOL SPRAY**
SONNENSCHUTZ-AEROSOLSPRAY
SPRAY AEROSOL SOLAIRE

(30) Priority: 24.03.2005 AU 2005901486
(43) Date of publication of application: 19.12.2007
(73) Proprietor: Ensign Laboratories PTY LTD, Mulgrave VIC 3170 (AU)
(72) Inventor: HOUGAZ, Louis, Wheelers Hill, Victoria 3150 (AU)
(74) Representative: V.O.
(86) International application number: PCT/AU2006/000405
(87) International publication number: WO 2006/099687

(56) References cited:
- WO-A1-00/66076
- WO-A1-01/05366
- DE-A1- 10 245 727
- JP-A- 1 117 817
- US-A- 4 686 099
- US-A1- 2004 151 673
- US-B1- 6 299 900
- PATENT ABSTRACTS OF JAPAN vol. 2003, no. 12 & JP 2004 224706 A (SHISEIDO CO LTD) 12 August 2004

## Description

### FIELD OF THE INVENTION

The present invention relates to sunscreen compositions containing at least one active sunscreen agent or UV radiation filter for providing protection for parts of the body against damage and/or injury caused by the sun. Embodiments of the present invention relate to sunscreen compositions containing a sunscreen agent or UV radiation filter dispensed from a container in the form of an aerosol and/or dispensed from an aerosol container for application to the skin of a person. Even more particularly, the present invention relates to sunscreen compositions containing a sunscreen agent that is active against the damaging effects of the sun in which the sunscreen compositions are dispensed from the container in either the form of an aerosol spray or from an aerosol container, such as a can, in the form of a spray, particularly a spray of a fine mist or similar. The present invention finds particular application as a sunscreen composition containing one or more active sunscreen agents incorporated into an emulsion that can be dispensed from an aerosol container in the form of a fine spray or mist, particularly a substantially consistent droplet size mist or spray so as to deliver the active sunscreen agent or agents to a person or animal in the form of a substantially even film containing the sunscreen agent or agents on to the skin of the person or animal so as to provide a substantially uniform coverage of the sunscreen agent or agents over the surface of the skin to which the sunscreen is applied thereby protecting the skin against damage from the sun. The present invention relates not only to aerosol sunscreen compositions having one or more active sunscreen agents and one or more propellants in the form of an emulsion but also to aerosol containers containing the sunscreen emulsion compositions, to methods of dispensing the sunscreen compositions from the aerosol container and to methods of applying the sunscreen agent or agents to parts of the body, such as to the skin, hair or the like.

Although the present invention will be described with particular reference to one form of the aerosol sunscreen composition, it is to be noted that the scope of the present invention is not limited to the described embodiments, but rather the scope of the present invention is broader by including other components of the composition, variations of the composition, other containers, other methods and forms of dispensing the formulations and compositions from the various containers and variations in methods of applying the different compositions to the various parts of the body of a person or animal and the like.

Also, even though the present invention is described primarily with reference to sunscreen formulations or compositions for applying to the skin of a person to provide protection against the harmful effects of the sun, it is to be noted that such sunscreen formulations or compositions can be applied to other parts of the body, such as, for example, to the hair, and the like. Also, the sunscreens can be used on humans and animals to provide protection against the harmful effects of the sun. Thus, the use of terms such as 'skin', 'person' and the like are meant to be non-limiting in scope and such terms are used for the sake of clarity and ease of expression rather than to delimit the scope of the invention.

It is to be noted that the word "sunscreen agent" is used in the specification for clarity of understanding and ease of expression. Its use is not meant to be limiting but its scope includes materials variously described as sunscreen agents, actives, UV filters, sun blocking agents, block out agents, sun protection agents and the like.

### BACKGROUND OF THE INVENTION

Sunscreen compositions are applied to the skin of a person to protect the skin from the harmful effect of the sun's rays so as to reduce or prevent the effects of sunburn and the like, particularly from harmful ultraviolet radiation, such as short wavelength UV-B radiation of from about 290 - 320 nm and from longer wavelength UV-A radiation of from about 320 - 400 nm wavelength, both of which are damaging to the skin. Exposure to the sun can cause reddening of the skin, sunburn and other temporary discomfort and can lead to long term damage to the skin. Excessive or prolonged exposure to sunlight, particularly to high intensity radiation from the sun, can lead to the development of skin disorders including serious conditions such as melanoma, basal cell carcinoma, and squamous cell carcinoma, local and systemic immune suppression, and the like. Conditions such as solar keratoses, sunburn, erythema, or redness of the skin are also manifestations of the effects of both short term and long term exposure to UV radiation, and with high exposure doses the skin will become painful and edematous and blistering may occur. Excessive exposure may also promote photosensitization or phototoxicity caused by endogenous factors, or by exposure to endogenous factors, such as certain plants, chemicals and the like. Even if excessive exposure to sunlight does not cause any medical condition, prolonged exposure results in photo-damage which is associated with premature ageing of the skin and is characterised by wrinkled, cracked or dry skin, such as for example skin having the characteristic appearance of "crocodile skin", sagging, loss of elasticity, mottled pigmentation or aged lines and the like. Even if such conditions are not harmful, they are unsightly. The major causes of premature aging of the skin, appear to include cumulative lifetime exposure to UV radiation having wavelengths in the region of from about 290 to 400 nm. Accordingly, sunscreen compositions have been developed to provide protection for the skin of animals and humans against long term damage from the sun and as a preventative against the conditions mentioned above.

One problem associated with some forms of existing sunscreen compositions is the unevenness of distribution of the sunscreen actives. Sunscreen formulations are usually applied topically as a relatively viscous lotion, cream or similar which must be spread over the entire surface of the skin, such as by rubbing or the like in order to spread the active sunscreen agent present in the composition over the skin to form a thin film or coating over the skin to provide protection. Rubbing the lotion or cream onto and over the skin in this manner results in an uneven distribution of the active sunscreen agent or agents in that there are some areas of high coverage with relatively greater film thickness having more sunscreen than is required and other areas of low coverage of relatively lower film thickness in which there is insufficient sunscreen agent applied to satisfactorily protect the skin. If the layer is too thick, it is wasteful of the sunscreen since such a thick layer is not necessary to provide adequate protection and if the layer is too thin, insufficient protection is provided to the underlying skin resulting in damage to the skin. As the coverage of the sunscreen over the surface is variable, the protection afforded by the sunscreen to the underlying skin is variable and the coverage is patchy which can result in skin located beneath the relatively thin coverage of sunscreen lotion being burnt and damaged as evidenced by the reddening appearance of the skin. Thus, with existing formulations, it is difficult to provide a consistent coverage of sunscreen agent. Therefore, there is a need to provide a sunscreen composition in a form that can be applied more evenly and for a method of applying the sunscreen to provide more uniform coverage of sunscreen on the skin of a person to provide the required protection.

Another problem associated with existing sunscreen formulations relates to the spreadability of the formulation, particularly to its ease of spreadability. Many existing formulations are difficult to apply, particularly in order to provide consistent protection for the entire surface of the skin. Reasons for the lack of spreadability include the physical form in which the sunscreen formulation is manufactured, such as for example, in the form of a lotion, cream, paste or similar high viscosity formulation, which is difficult to apply and spread evenly over the surface of the skin. Formulations at the other end of this viscosity spectrum, being very thin liquid such as for example, liquids having almost the viscosity of water, do not have sufficient "cling" properties to adhere to the skin but rather have a tendency to "run off" the surface of the skin before the formulation can be spread evenly over the surface of the skin. This run off leads to wastage of the sunscreen and to reduced or uneven protection of the skin.

Accordingly, it is an object of the present invention to provide a sunscreen composition in a form that can be dispensed more readily from a container to deliver a substantially uniform film or to provide a substantially uniform coverage of the sunscreen agent on the surface of the skin in order to provide more consistent protection for the skin. In one embodiment, this object is achieved by incorporating the sunscreen agent or agents into a suitable composition or formulation contained in an aerosol container and dispensing the sunscreen aerosol spray composition from the container in the form of a fine mist, spray or similar to provide a uniform coverage of the composition thereby providing uniform protection.
DE10245727 describes W/O (water in oil) emulsions with one or more lipophilic propellant gases that can be sprayed as aerosol and which can contain UV filters.
JP 2004/224706 mentions O/W emulsion compositions containing a UV absorber packed in an aerosol container wherein the propellant is compressed gas.

### SUMMARY OF THE INVENTION

The invention is the aerosol container including a sunscreen composition and dimethyl ether as propellant as defined in claim 1.

According to one aspect of the present invention there is provided a sunscreen composition for providing protection against the effects of the sun on the skin of the person to whom the composition is applied, said composition comprising a base sunscreen emulsion including one or more sunscreen agents for providing protection to the skin against the harmful effects of the sun, and a propellant in the emulsion, for facilitating dispensing of the sunscreen composition from a container wherein the sunscreen composition is capable of being dispensed in the form of an aerosol spray from the container using the propellant to produce a substantially consistent spray of the sunscreen composition for applying a substantially uniform film of the sunscreen composition to the skin thereby providing a more uniform protection against the damaging effects of the sun.

According to another aspect of the present invention, there is provided a sunscreen composition containing one or more sunscreen agents for providing protection against the effects of the sun on the skin of a person to whom the sunscreen composition is applied, said sunscreen composition being in the form of an emulsion that is capable of being dispensed from an aerosol container using a propellant contained within the container, such that the sunscreen composition is dispensed in the form of a fine consistent spray mist or similar for applying a substantially uniform film of the sunscreen composition to the skin of the person to whom the sunscreen is applied thereby providing substantially uniform protection against the harmful effects of the sun.

According to another aspect of the present invention there is provided an aerosol container containing a sunscreen composition having one or more sunscreen agents for providing protection against the effects of the sun to the skin of the person to which the composition is applied, said container being an aerosol container having a dispensing valve and containing a sunscreen composition in the form of an emulsion comprising at least one sunscreen agent and at least one propellant such that in response to operation of a dispensing valve of the container the propellant dispenses the sunscreen composition from the container in the form of a substantially consistent spray, mist or similar facilitating application of a substantially uniform film of the sunscreen composition to the skin of the person so as to provide a substantially even coverage of the sunscreen composition on the skin to provide more consistent protection against the sun.

According to another aspect of the invention, there is provided a method of applying to the skin of a person, a sunscreen composition comprising one or more sunscreen agents in the form of an emulsion to prevent or reduce the harmful and/or damaging effects of the sun on the skin comprising using a propellant or blend of propellants to dispense the sunscreen composition from an aerosol container in response to operation of a dispensing valve provided on the container, said sunscreen composition being in the form of an essentially consistent spray for applying a substantially uniform film of the spray containing the sunscreen agents to the skin of the person so as to produce a substantially uniform coverage of the skin thereby protecting the skin against the effects of the sun.

It is to be noted that the use of the term 'person' in the present specification is not limited to humans but includes animals as well.

### DETAILED DESCRIPTION OF THE INVENTION

Typically, the spray of sunscreen composition has a cooling effect on the skin. More typically, the cooling effect is enhanced by evaporation of one or more components of the sunscreen formulation or composition. Even more typically, the components that evaporate are at least some of the non-sunscreen agents in the composition so that the sunscreen agents or filters remain on the skin after evaporation. Even more typically, the one or more propellants of the emulsion evaporate or boil on discharge from the container to provide the cooling effect on the skin. Even more typically, the boiling of the propellant droplets or particles causes other components of the composition to break up i.e. disintegrate thereby facilitating more even distribution of droplets of the composition in the form of a fine mist or spray.

Typically, the sunscreen composition contains one or more, and preferably many components for a variety of purposes. More typically, the sunscreen composition includes at least one sunscreen agent, an emulsifier, a stabilizer, a neutraliser, and a propellant base, including at least one propellant. More typically the sunscreen composition further comprises an emollient, a humectant, a preservative, an antioxidant, water and the like. Other additives may be added to the sunscreen composition as required or desired. Additionally, two or more of each type of additive may be added to the composition such as for example, two or more emulsifiers and the like.

Typically, the composition includes a sunscreen base mixture that contains the at least one sunscreen agent. More typically, the amount of sunscreen base mixture in the total of the composition is in the range of about 1% to about 90%, preferably in the range from about 20% to 80%, more preferably in the range from about 40% to about 70% and most preferably in the range from about 50% to 65% of the weight of the total composition.
The container is capable of containing one or more propellants, is a pressurized container, and is an aerosol container.

Typically, the aerosol container dispenses a spray, typically an aerosol spray made up of a composition of active ingredients and additives. Typically, the additives include excipients or other vehicles or the like in the form of an emulsion and a propellant or blend of propellants.

Typically the sunscreen formulation is an emulsion or an emulsion/suspension, or an emulsion and suspended particles. The emulsion is an oil-in-water emulsion or a water-in-oil-in-water emulsion.
More typically, the non-aqueous
phase of the emulsion is a solvent, typically, an organic solvent, or any other hydrophobic material and includes materials such as oils, waxes, waterproofers, film formers, emollients and the like. Typical examples of the hydrophobic material include esters, silicones, oils, synthetic or natural waxes and the like.

Typically, the emulsion includes intimate contact between the sunscreen agent or agents and the propellant or propellants.

Typically, the sunscreen composition of the present invention is one phase, two phases or multiphase.
Typically, compositions of the present invention can have any viscosity from about 10 mPa.s (cps) to about 500,000 mPa.s (cps), preferably from about 100 mPa.s (cps) to about 10,000 mPa.s (cps), more preferably from about 300 mPa.s (cps) to about 5,000 mPa.s (cps), all measured at 21 °C. The state of the composition can be from being almost gelatinous to being extremely thin.

Typically, the propellant is a liquefied propellant under pressure. More typically the liquefied propellant is responsible for and/or produces a fine, even and consistent spray, particularly on being discharged from the container. More typically, the propellant converts into a gaseous phase on leaving the container at the point of discharge from the container. Typically, the propellant aids in distribution of the sunscreen agent or agents in the mist or spray.

Typically, the propellant is a gas or mixture of gases, more typically a compressed gas or a mixture of gases. Typically, the gas is air or an air containing gas. More typically, the propellant is a volatile material, such as a material having a low boiling point that causes it to evaporate quickly. More typically, the propellant has a high volatility, particularly at room temperatures. Typically, the boiling points of the propellants vary from about -50°C to about 50°C.

Typically, the spray is in the form of a fine particle size or fine droplet size mist, fog, aerosol, spray or the like. The spray has a particle size or droplet size of from about 0.1 micron to about 5000 micron, preferably from about 0.2 micron to about 1000 micron, more preferably from about 1.0 micron to about 500 micron.

Typically the spray or mist delivers a fine even coverage of a film of the sunscreen composition to skin surfaces. More typically, the film of sunscreen is a film of the sunscreen agent or active. Even more typically, the sunscreen agent or active is mixed with excipients to form the sunscreen composition.

Typically the sunscreen base mixture is an emulsion. More typically the sunscreen base mixture includes sunscreen agents, emulsifiers, water proofing agent or agents, film formers, solvents, inorganic material, esters, perfumes, antioxidants, preservative materials or the like. Even more typically the sunscreen base includes a propellant base to be included in the emulsion system which, when packaged in an aerosol container, with a suitable valve dispensing system, will deliver a consistent and even spray pattern of sunscreen for application to the skin.

Typically the present invention relates to the combination of the sunscreen composition with propellant and an aerosol container in which the aerosol container has a dispensing valve. More typically, the dispensing valve is a customised tailored aerosol valve to dispense the composition in the form of a substantially uniform spray. Typically, the container has a valve. More typicallyl, the valve allows upright use, inverted use, or use in any direction or orientation. Typically, the valve includes a cup in which the cup of the valve can be of any suitable form and can be of different configurations. Typically, the mounting cup can be aluminium, coated aluminium, steel, coated or laminated steel or the like. Typically, the container has an actuator. The actuator can be one of many different possible configurations, types, orifice size, spray patterns or the like. Typically, the dispensing valve actuator has a standard dispensing orifice or a reverse taper orifice or the like. The valve and its components are selected to produce a suitable spray pattern of selected droplet size when spraying the intimate mixture of the sunscreen agents and propellants in direct contact with one another as the emulsion is sprayed from the aerosol container. The present invention also relates to a method of applying the sunscreen composition to the skin, particularly over a defined area of the skin so as to leave a substantially uniform film of sunscreen over the skin.

Typically, the container is a conventional aerosol container, or is a container made from one or more materials, such as for example, aluminium, steel, plastic, glass or the container can be lined with a different material from which the body of the container is made including combinations of different materials. The container is available in a number of different sizes, types, configurations, shapes or the like.

### SUNSCREEN AGENTS

Typically the sunscreen agents that can be used in the sunscreen composition of the present invention are capable of absorbing or blocking the harmful effects of the sun, particularly the ultra-violet radiation from the sun. More typically, blocking and/or absorbing radiation in the 290 to 400 nm wavelength region. More typically, the sunscreen agents are all agents that have been approved for use in sunscreen formulations by one or more regulatory authority. Suitable sunscreen agents include, for example, Para-aminobenzoic Acid (PABA), Benzophenone-1, Benzophenone-2, Benzophenone-3, Benzophenone-4, Benzophenone-5, Benzophenone-6, Benzophenone-8, Benzophenone-9, Benzophenone-12, Ethyl Dihydroxypropyl-PABA, Glyceryl PABA, Homosalate, Methyl Anthranilate, Octocrylene, Octyl Dimethyl PABA, Octyl Methoxycinnamate, Octyl Salicylate, 2-Phenylbenzimidazole-5-sulphonic acid, Triethanolamine Salicylate, 4-Methylbenzylidene Camphor, Red Petrolatum, and mixtures thereof. More typically the sunscreen agents are Octyl Methoxycinnamate,Ethylhexyl Methoxycinnamate, Isoamyl p-Methoxycinnamate, Cinoxate, Dioxybenzone, Homosalate, Ethylhexyl Salicylate, Para-aminobenzoic acid (PABA), Ethylhexyl Dimethyl PABA, PEG-25 PABA, Benzylidene camphor sulfonic acid, 3-Benzylidene camphor, 4-Methyl Benzylidene camphor, terephtalylidene dicamphor sulfonic acid, polyacrylamidomethyl benzylidene, phenylbenzimidazole sulfonic acid, 2-Hyroxy-4-Methoxybenzophenone, octocrylene, menthyl anthranilate, ethylhexyl triazone, diethylhexyl butamido triazone, butyl methoxydibenzoylmethane, camphor benzalkonium methosulfate, methylene bis-benzotriazolyl tetramethyl butylphenol, monosodium salt of 2,2 bis-(1,4-phenylene) 1H-benzimidazole-4,6-disulfonic acid, (1,3,5)-Triazine-2,4-bis-((4-(2-ethylhexyloxy)-2-hydroxy)-phenyl)-6-(4-methoxyphenyl), polysilicone 15, DEA methoxycinnamate, digaloyl trioleate, glyceryl PABA, isopropylbenzyl salicylate, triethanolamine salicylate, glyceryl ethylhexanoate di-methoxycinnamate, methyl diisopropyl cinnamate,isopropyl methoxy cinnamate diisopropyl cinnamate esters mixture, benzophenone-9, trolamine salicylate, ethylene glycol salicylate, dipropylene glycol salicylate, methyl salicylate, phenyl salicylate, ethyl PABA (benzocaine), glyceryl PABA, amyl dimethyl PABA, butyl methoxy PABA, phenylbenzimidazole sulfonic acid, terephthalylidene dicamphor sulfonic acid, drometrizole trisiloxane silatrizole, camphor benzalkonium methosulphate, Hososalate, Phenylbenz-imidazole sulphonic acid, Terephthalylidene dicamphor sulphonic acid, Butyl methoxydibenzoy 1 methane, Benzylidene camphor sulfonic acid and salts, Octorylene, Polyacrilamido-methyl benzylidene camphor, Octyl methoxy-cinnamate, PEG-25-PABA, Isoamyl p methoxy-cinnamate, Octyltriazone, Drometrizole trisiloxane, Dioctyl butamido triazone, 4-methylbenzylide ne camphor, 3-Benzylidene camphor, Octyl Salicylate, Octyl dimenthyl PABA, Benzophenone 4, Benzophenone 5, Methylene bix-benzotriazolyl tetramethyl butylphenol, Bisymidazylate, Anisotriazine, Polysilicone-15, Titanium dioxide including mixtures thereof.

The sunscreen agents included in the sunscreen composition of the present invention are present in an amount of about from 1 wt.% to about 40 wt.% of the total weight of the composition of the invention. It is to be noted that the amount of sunscreen agent in the composition will be in accordance with the required sun protection factor (SPF) and/or UV filtering required of the sunscreen composition. Usually, the higher the SPF, and UV filtering, the greater the amount of sunscreen agents incorporated into the sunscreen formulation. Preferably, the amount of sunscreen agent or agents (UV filters) included in the sunscreen composition including propellant is from about 0 wt.% to about 30 wt. depending upon the SPF required and also to achieve the necessary level of UVA protection.

Typically, the sunscreen composition has any SPF factor, such as for example from about 2 to 100, more typically about 10 to 40 or the like as required in accordance with the regulations in force in the country in which the composition is to be used.

Typically, the sunscreen agents or active agents used in the present invention include a wide variety of different types of sunscreen agents, such as, sunscreen agents functioning by reflecting, blocking, absorbing or scattering UV radiation or other mechanism of filtering or a combination of these effects.

One type of active agent includes chemical absorbers. With this type of chemical compound the sunscreen agent or active is excited to a higher energy state from its ground state by absorbing UV radiation. As the excited molecule in the higher energy level returns to the ground state energy is emitted. The emitted energy is lower in magnitude and longer in wavelength than the energy that initially caused the excitation of the molecule. The wavelength of the emitted radiation is in the infrared region of the electromagnetic spectrum. However, the shift in wavelength to the infrared region and the lessening in intensity result in there being little or no damage to the skin caused by the sun and hence chemical absorbers act as good sunscreening agents. Other types of sunscreen agents include combinations of both physical and chemical filters. Some embodiments of the sunscreen formulation of the present invention may contain one or more individual sunscreen agents such as combinations of one, two, three, four, five, six or more individual sunscreen agents or types of sunscreen agents functioning in different ways. In one embodiment there may be three different sunscreen agents whilst in still others there may be four or five different sunscreen agents to provide consistent protection over almost all of the UV spectrum since individual suncreeen agents have maximum protection at different points in the entire spectrum of radiation and have effective sunscreen protection over different ranges within the spectrum. As an example, one sunscreen agent may provide maximum protection at one end of the UV region whilst another may provide maximum protection at the other end of the spectrum, whilst a third or fourth will provide maximum protection at different regions within the ends of the spectrum of the ultra violet region so that all the sunscreen agents contribute to providing a broad spread of protection over the entire region required even though individual sunscreen agents may provide protection for a small range within the overall spectrum only. Also, some agents may provide effective protection over a narrow band width whereas others provide protection over a wide band width within the overall spectrum. By mixing and matching the protection characteristics, it is possible to formulate a sunscreen formulation with acceptable protection over a broad UV spectrum.

### PROPELLANTS

The propellant used in the present invention is dimethyl ether. The propellants are preferably in intimate contact with the sunscreen agent or agents in the emulsion and are not located separately from the emulsion containing the sunscreen agent.
suitable propellants or mixture of propellants can also be

These are contained in the system at pressures between 5 and 200 psig, more specifically between 20 and 100 psig and most specifically between 30 and 80 psig.

### EMULSIFIERS

The sunscreen composition of the present invention is in the form of an emulsion. Typically, the emulsion can be made using emulsifiers or can be emulsifier-free by being made without using emulsifiers.
The emulsion is an oil in water emulsion or a water in oil in water emulsion.
Additionally, the emulsion can be a standard emulsion, a nano-emulsion or a micro-emulsion. More typically, the emulsion of whichever type has a particle size of from about .001 to 1000µ, preferably from about 0.5 to 1000µ depending upon the type of emulsion.

Typically, the emulsion includes an emulsifier to promote the formation of the emulsion. More particularly the emulsifier assists in stabilization of the emulsion and of the sunscreen composition. The emulsifiers that can be used in the present invention include any suitable emulsifier, such as for example, sorbitan oleate, sorbitan sesquioleate, sorbitan isostearate, sorbitan trioleate, polyglyceryl-3-diisostearate, polyglycerol esters of oleic/isostearic acid, polyglyceryl-6 hexaricinolate, polyglyceryl-4-oleate, polygylceryl-4 oleate/PEG-8 propylene glycol cocoate, oleamide DEA, sodium glyceryl oleate phosphate, hydrogenated vegetable glycerides phosphate PEG-20 stearate, PEG-30 dipolyhydroxystearate, PEG-40 hydrogenated castor oil, PEG-60 hydrogenated castor oil, PEG-40 stearate, polyglyceryl-3 methylglucose di stearate, polysorbate 20, polysorbate 60, polysorbate 80, potassium cetyl phosphate, DEA cetyl phosphate, ethomeen, dimethicone PEG-7 phosphate, and mixtures thereof.

It is to be noted that emulsifiers other than those listed above can be used in the present invention either alone or in combination with other emulsifiers depending upon requirements.

Typical amounts of emulsifiers included in the emulsion and/or in any one of the phases of the emulsion include the following: from about 0.10% to 20.0%, preferably from about 0.5% to 10.0% and more preferably from about 1.0% to 5.0% of the total weight of the composition.

Stable emulsion can also be manufactured under certain conditions without containing emulsifiers.

### FILM FORMERS

Typically, film formers are included in the sunscreen composition to assist in forming a film when the sunscreen is in contact with the skin. More typically the film forming materials in the sunscreen formulation form a substantially continuous film. Even more typically the substantially continuous film is substantially uniform in thickness providing a substantially uniform coverage of the skin without significant variation in the thickness of the film thereby avoiding patches or blotches of sun damaged skin owing to too little coverage of the sunscreen composition over the skin. Typically the film formers included in the composition are water proofers, waxes and silicones. Typically, examples of waterproofers or film formers include the following: polyethylene polymers, beeswax, butylenes/ethylene/styrene copolymers, stearates, silicones, paraffinium liquidum, PVP/hexadecane copolymer, tricontanyl PVP, dimethicone, cyclomethycones, dimethicone copolyol, or the like, including combinations or two or more.

Typically, the film formers are present in an amount of from 0.10% to 20.0%, preferably 0.5% to 5.0% of the total weight of the composition.

### NEUTRALISERS

Neutralisers are in the composition of the present invention to neutralize components such as the thickeners, emulsifiers and other additives of the sunscreen composition. Typical examples of neutralizers include hydroxides, amines, EDTA salts and the like. Other neutralizers include triethanolamine, sodium hydroxide, potassium hydroxide, ethomeen (PEG-15 cocamine), or the like including combinations or two or more.

More typically, further examples of the neutralizers include sodium hydroxide, potassium hydroxide, diethanolamine, triethanolamine, aminomethyl propanol, trisodium ethylenediaminetetraacetic acid, and mixtures thereof. A particularly preferred neutralizer is triethanolamine.

Typically the neutraliser or neutralisers are present in an amount of from about 0.1 wt.% to about 8 wt.% of the weighty of the sunscreen composition of the present invention. More typically, the neutralizer(s) are present in amount from about 1 wt.% to about 5 wt.% preferably from about 0.10% to about 2.0% of the total weight of the composition.

### THICKENERS

Thickeners are a typical example of additives added to the sunscreen composition. Examples of thickeners include carbomers, xanthan gum, celluloses, acrylates/cross polymers or the like, including combinations or two or more.

It is to be noted that the thickener is included to adjust the viscosity of the sunscreen formulation to assist in providing a fine mist of spray and/or forming a uniform coverage of the sunscreen formulation on the skin.

The amount of thickener added to the compositions include from about 0.01% to 20.0%, preferably from about 0.01% to 10.0% of the total weight of the composition.

### EMOLLIENTS

The present composition may additionally contain one or more emollients. An emollient provides a softening or soothing effect on the surface of the skin and is generally regarded as being non-toxic and hence is considered safe for topical use, particularly topical use in the form of a uniform film of sunscreen. Emollients also help control the rate of evaporation and the tackiness of the composition. Typical examples of emollients include mineral oils, esters, natural oils, lanolin derivatives and the like. Examples of emollients include: glycerine, sorbitol, propylene glycol, isopropyl myristate, isopropyl palmitate, butylene glycol, caprys caprylic triglyceride, coco-caprylate/caprate, cocoglycerides, or the like including combinations of two or more.

More typically the emollients include mineral oil, lanolin oil, coconut oil, cocoa butter, olive oil, aloe extracts, jojoba oils, castor oil, fatty acids such as oleic and stearic, fatty alcohols such as cetyl and hexadecyl, diisopropyl adipate, hydroxybenzoate esters, benzoic acid esters, particularly C10-C16 alcohols, isononyl iso-nonanoate, alkanes such as mineral oil, silicones such as dimethyl polysiloxane, ethers such as polyoxypropylene butyl ethers and polyoxypropylene cetyl ethers, and C12-C15 alkyl benzoates including mixtures of two or more of the above.

Typically the emollient is present in an amount of from about 0.1 % to about 20.0% of the total weight of the sunscreen composition. The preferred amount of emollient is from about 0.1% to 20.0%, more preferably from about 1.0% to 10.0% of the total weight of the composition.

### PRESERVATIVES

Additives including preservatives, antioxidants and similar materials can be incorporated into the sunscreen composition of the present invention. Typical examples of such preservatives or antioxidants include the parabens. Other examples include the following: vitamin E, vitamin E acetate, vitamin C, butylated hydroxytoluene, methylparaben and mixtures thereof. One or more preservatives/antioxidants may be present in an amount of about 0.01 wt% to about 2 wt.% of the total weight of the present invention. Preferably, one or more preservatives/antioxidants are present in an amount about 0.1 wt.% to about 1 wt.%.

Typically, preservatives are added to the sunscreen formulation of the present invention in order to preserve the formulation against deterioration and/or degradation including microbial growth or the like.

Examples of preservatives include: BHA, BHT, phenoxyethanol, methyldibromo glutaro-nitrile, potassium sorbate, propylparaben, methylparraben, butylparraben, ethylparraben, imidazolidinyl urea, and others including mixtures of two or more.

### HUMECTANTS

A moistening agent, such as a humectant, may be incorporated into the composition of the present invention. Suitable humectants include glycerin, polyethylene glycol, polypropylene glycol, sorbitol including mixtures thereof.

### RHEOLOGICAL ADDITIVES

The sunscreen compositions of the present invention can include a rheological additive, such as for example magnesium aluminium silicate, hydroxypropyl cellulose, carboxymethyl cellulose, carbomer, or the like.

### BRIEF DESCRIPTION OF METHODS OF MANUFACTURE

The process used to manufacture the sunscreen composition of the present invention includes the following steps.

Typically the sunscreen composition is introduced into the aerosol container. More typically, the aerosol sunscreen composition is added to the aerosol can and then the can is pressurised with the desired amount of propellant.

The sunscreen formulation of the present invention may be prepared by using techniques and methods well known to persons skilled in these arts. And the aerosol container can be made and filled with the sunscreen composition in accordance with well known methods for filling such containers.

Sunscreen compositions of the invention can be made by the following process or modifications of this process. Other methods for making sunscreen compositions of the invention are also possible. A first phase comprising the hydrophobic components including such components as emollients, dispersing agents, emulsifiers, and, optionally, antioxidants, and other additives, are combined together by stirring and heating at temperature of from about 10°C to about 90°C, preferably from about 55°C to about 90°C, and most preferably from about 70°C to 85°C, until all of the solid material of the components is dissolved. The sunscreen agent can be added to this mixture as it is being prepared or it can be added after the mixture is formed. A second phase comprising water, thickening agents, and other hydrophilic ingredients including, for example, humectants, emulsifiers, and preservatives can be formed by combining the individual compounds of this phase whilst stirring and heating at a temperature of from about 10°C to about 90°C, preferably from about 55°C to about 90°C, and most preferably at about 70°C to 85°C. After forming, the second phase is then slowly added to the first phase while stirring at high speed. The mixture of the first and second phases is then gently homogenized until the temperature of the mixture is between about 35°C to 40°C so as to form an emulsion. Preservatives are added, and the emulsion is then stirred, preferably with an anchor mixer, and allowed to reach 25-30°C before packaging.

It is to be noted that the emulsion may be emulsifier free or it can be made cold with or without homogenization or without excessive energy input. The emulsion can be made in any other suitable alternative manner to achieve stability and variable particle size, including nano- and micro-emulsion.

Typically the aerosol container is made from tin plate with appropriate internal lining. Alternatively the container may be aluminum with appropriate internal lining or a composite of different materials, including plastics, PET, polystyrene and other suitable materials.

It is to be noted that filling and pressurizing of the container can be done in any suitable manner including pressure filling or under the cup filling or the like.

The containers can be cold filled, use pressure filling, rotary pressure filled, or undercap filled.

The compositions of the present invention may be applied with any device having an aerosol spraying means, such as an aerosol container.

### DETAILED DESCRIPTION OF EMBODIMENTS OF THE INVENTION

The present invention will now be described by way of example with reference to the following non limiting example which illustrates the typical formulation of one embodiment of the present invention. It is to be noted that the ranges of the individual components listed in Column X of Table 1 illustrate typical ranges of values of the amounts by weight of the components that can be selected for incorporation into compositions in accordance with the present invention. Further, it is to be noted that the ranges of values of amounts indicated in Column Y of Table 1 illustrate more specific prepared amounts of the components that can be used in preparing formulations in accordance with the present invention.

In one embodiment, the composition of the present invention is a sunscreen composition containing one or more sunscreen agents. In another embodiment the sunscreen composition includes one or more moisturizing agents or is a moisturizing composition containing one or more sunscreen agents.

In other embodiments, the sunscreen composition includes one or more artificial tanning actives or accelerators, such as for example, dihydroxy acetone (DHA) or is a tanning composition containing one or more sunscreen agents. In another embodiment, the composition includes one or more moisturizing agents with one or more tanning actives or accelerators as well as one or more sunscreen agents.

In other embodiments, the sunscreen composition includes one or more skin whitening agents or is a skin whitening agent with one or more sunscreen agents.

In other embodiments, the composition may be a combination of all of the above compositions or agents.

In other embodiments, the compositions may have water resistance or may not have water resistance.

**TABLE 1**

| | **COLUMN X** | **COLUMN Y** |
|---|---|---|
| **PART A (Oil Phase)** | | |
| **%w/w** | | **More specifically % w/w** |
| 0 - 30 | Solvent | 5 - 20 |
| 0 - 10 | Film former | 0.5 - 10 |
| 0 - 10 | Fatty acid | 1.0 - 8.0 |
| 0 - 10 | Emulsifier | 0.1 - 3.0 |
| 0 - 20 | Waterproofer | 0.1 - 5.0 |
| 0 - 30 | UV Filters | 5.0 - 20.0 |
| 0-5 | Antioxidant | 0.01 - 0.5 |
| 0 - 10 | Wax | 1.0 - 8.0 |
| 0-2 | Preservative | 0.1 - 1.5 |

| PART B (Water Phase) | | |
|---|---|---|
| %w/w | | More specifically % w/w |
| 0 - 90 | Water | 20 - 70 |
| 0 - 40 | Humectant | 5 - 20 |
| 0 - 15 | Thickener | 0.1 - 5.0 |
| 0 - 15 | Neutraliser | 0.1 - 2.0 |
| 0 - 10 | Emulsifier | 0.1 - 5.0 |
| 0 - 5 | Sequestering agent | 0.01 - 2.0 |

| Part C (Preservatives) | | |
|---|---|---|
| 0 - 10 | Preservatives | 0.1 - 2.0 |

| Part D (Fragrance) | | |
|---|---|---|
| 0 - 5 | Fragrance | 0.05 - 2.0 |

| Part E (Propellants) | | |
|---|---|---|
| 0 - 60 | Propellants | 2.0 - 50.0 |

### ADVANTAGES OF THE INVENTION

It is to be noted that an integral part of the composition and system of the present invention is that the composition can be in the form of an emulsion which contains the sunscreen agent and propellant so that there is contact between the sunscreen agent and propellant, preferably intimate contact between the sunscreen agent and propellant in the emulsion in the container so that the sunscreen agent and propellant are in direct contact with each other and are not separated from each other or are not stored in different directions within the container or in different compartments of the container.

The propellant being in direct contact with and being part of the emulsion system is responsible for the improved spray pattern and breakup of the composition as the composition is being discharged from the container. The improved spray pattern being more uniform and consistent in both droplet size and distribution to form a fine mist or spray, results from the droplets of propellant effectively boiling upon being discharged from the container which in turn assists in disintegrating the droplets of the other components of the composition, particularly the sunscreen agents, to provide improved distribution of fine droplets of the sunscreen agents in the form of a fine mist or spray of more uniform droplet size. This in turn allows a more even coverage of the skin by the sunscreen agents.

Additionally, the improved breakup of the composition caused by the boiling of the propellant in the emulsion promotes a cooling effect on the skin because of the more even distribution.

A further advantage of the present invention is that the container and contents are cheaper to manufacture since a single compartment container can be used for containing the emulsion containing the sunscreen agent and the propellant, rather than having to separately store the emulsion and propellant individually as required in many other systems, such as for example, systems having the bag in can, bag on valve, piston in can or other systems requiring two or more separate compartments or storage areas for the emulsion and propellant.

A further advantage of the present invention includes that the container may be used in any orientation or direction since the propellant is contained within the emulsion along with the sunscreen agent.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

It will be understood to persons skilled in the art of the invention that many modifications may be made without departing from the spirit and scope of the invention.

## Claims

1. An aerosol container capable of containing one or more propellants, including a sunscreen composition and at least one propellant, said propellant being dimethyl ether, said sunscreen composition providing protection against the effects of the sun on the skin of a person to whom the composition is applied, wherein the sunscreen composition is an emulsion containing at least one sunscreen agent, the sunscreen agent and propellant being in direct contact with each other in the emulsion and not separated from each other, the container being pressured at a pressure of from 3.4x10⁴ Pa to 5.9x10⁵ Pa (20 to 100 psig), the at least one sunscreen agent being effective to provide protection to the skin against the harmful effects of the sun when in contact with the skin of a person, the sunscreen composition being dispensable from the container in the form of a spray having a droplet size of from 0.01 µm (micron) to 5000 µm (micron), the sunscreen composition being effective to form a substantially continuous film when applied to the skin of the person wherein when the emulsion is dispensed from the container, the sunscreen agent forms particles which, on contact with the skin of the person, form a substantially continuous film of uniform thickness, wherein the emulsion is an oil in water or a water in oil in water emulsion.

2. An aerosol container according to claim 1, wherein the amount of sunscreen agent in the emulsion is from 1% to 90-%, preferably from 20% to 80%, more preferably from 40% to 70%, and most preferably from 60% to 65% of the weight of the sunscreen composition.

3. An aerosol container according to any preceding claim, wherein the container is at a pressure of between 1.0x10⁵ and 4.5x10⁵ Pa (30 and 80 psig).

4. An aerosol container according to any preceding claim wherein the spray has a droplet size of from 1 µm (micron) to 50 µm (micron).

5. An aerosol container according to any preceding claim wherein the sunscreen agent is Para-aminobenzoic Acid (PABA), Benzophenone-1, Benzophenone-2, Benzophenone-3, Benzophenone-4, Benzophenone-5, Benzophenone-6, Benzophenone-8, Benzophenone-9, Benzophenone-12, Ethyl Dihydroxypropyl-PABA, Glyceryl PABA, Homosalate, Methyl Anthranilate, Octocrylene, Octyl Dimethyl PABA, Octyl Methoxycinnamate, Octyl Salicylate, 2-Phenylbenzimidazole-5-sulphonic acid, Triethanolamine Salicylate, 4-Methylbenzylidene Camphor, Red Petrolatum, Octyl Methoxycinnamate, Ethylhexyl Methoxycinnamate, Isoamyl p-Methoxycinnamate, Cinoxate, Dioxybenzone, Homosalate, Ethylhexyl Salicylate, Para-aminobenzoic acid (PABA), Ethylhexyl Dimethyl PABA, PEG-25 PABA, Benzylidene camphor sulfonic acid, 3-Benzylidene camphor, 4-Methyl Benzylidene camphor, terephtalylidene dicamphor sulfonic acid, polyacylamidomethyl benzylidene, phenylbenzimidazole sulfonic acid, 2-Hydroxy-4-Methoxybenzophenone, octocrylene, menthyl anthranilate, ethylhexyl triazone, diethylhexyl butamido triazone, butyl methoxydibenzoylmethane, camphor benzalkonium methosulfate, methylene bis-benzotriazolyl tetramethyl butylphenol, monosodium salt of 2,2 bis-(1,4-phenylene) 1H-benzimidazole-4, 6-disulfonic acid, (1,3,5)-Triazine-2,4-bis-((4-(2-ethylhexyloxy)-2-hydroxy)-phenyl)-6-(4-methoxyphenyl), polysilicone 15, DEA methoxycinnamate, digaloyl trioleate, glyceryl PABA, isopropylbenzyl salicylate, triethanolamine salicylate, glyceryl ethylhexanoate dimethoxycinnamate, methyl diisopropyl cinnamate, isopropyl methoxy cinnamate diisopropyl cinnamate esters mixture, benzophenone-9, trolamine salicylate, ethylene glycol salicylate, dipropylene glycol salicylate, methyl salicylate, phenyl salicylate, ethyl PABA (benzocaine), glyceryl PABA, amyl dimethyl PABA, butyl methoxy PABA, phenylbenzimidazole sulfonic acid, terephthalylidene dicamphor sulfonic acid, drometrizole trisiloxane silatrizole, camphor benzalkonium methosulpate, Hososalate, Phenylbenz-imidazole sulphonic acid, Terephthalylidene dicamphor sulphonic acid, Butyl methoxydibenzoy 1 methane, Benzylidene camphor sulfonic acid and salts, Octorylene, Polyacrilamido-methyl benzylidene camphor, Octyl methoxy-cinnamate, PEG-25-PABA, Isoamyl p methoxy-cinnamate, Octyltriazone, Drometrizole trisiloxane, Dioctyl butamido triazone, 4-methylbenzylide ne camphor, 3-Benzylidene camphor, Octyl Salicylate, Octyl dimethyl PABA, Benzophenone 4, Benzophenone 5, Methyl bixbenzotriazolyl tetramethyl butylphenol, Bisymidazylate, Anisotriazine, Polysilicone-15, or mixtures of two or more thereof.

## Patentansprüche

1. Aerosolbehälter, der geeignet ist, ein oder mehrere Treibgas(e) zu enthalten, einschließlich einer Sonnenschutzzusammensetzung und mindestens ein Treibgas, wobei das Treibgas Dimethylether ist, wobei die Sonnenschutzzusammensetzung Schutz gegen die Auswirkungen der Sonne auf die Haut einer Person, bei der die Zusammensetzung angewendet wird, bietet, wobei die Sonnenschutzzusammensetzung eine Emulsion ist, die mindestens ein Sonnenschutzmittel enthält, wobei das Sonnenschutzmittel und Treibgas in direktem Kontakt miteinander in der Emulsion sind und nicht voneinander getrennt sind, wobei der Behälter einem Druck von 3,4x10⁴ Pa bis 5,9x10⁵ Pa (20 bis 100 psig) ausgesetzt ist, wobei das mindestens eine Sonnenschutzmittel wirksam ist, um Schutz für die Haut gegen die schädlichen Auswirkungen der Sonne zu bieten bei Kontakt mit der Haut einer Person, wobei die Sonnenschutzzusammensetzung aus dem Behälter in Form eines Sprays mit einer Tröpfchengröße von 0,01 µm (Mikron) bis 5000 µm (Mikron) abgegeben werden kann, wobei die Sonnenschutzzusammensetzung wirksam ist, um einen im Wesentlichen kontinuierlichen Film zu bilden beim Anwenden auf der Haut der Person, wobei, wenn die Emulsion aus dem Behälter abgegeben wird, das Sonnenschutzmittel Partikel bildet, die bei Kontakt mit der Haut der Person einen im Wesentlichen kontinuierlichen Film von gleichmäßiger Dicke bilden, wobei die Emulsion eine Öl-in-Wasser- oder eine Wasser-in-Öl-in-Wasser-Emulsion ist.

2. Aerosolbehälter nach Anspruch 1, wobei die Menge von Sonnenschutzmittel in der Emulsion von 1 % bis 90 %, bevorzugt von 20 % bis 80 %, bevorzugter von 40 % bis 70 % und am meisten bevorzugt von 60 % bis 65 % des Gewichts der Sonnenschutzzusammensetzung beträgt.

3. Aerosolbehälter nach einem der vorhergehenden Ansprüche, wobei der Behälter einem Druck zwischen 1,0x10⁵ und 4,5x10⁵ Pa (30 und 80 psig) ausgesetzt ist.

4. Aerosolbehälter nach einem der vorhergehenden Ansprüche, wobei das Spray eine Tröpfchengröße von 1 µm (Mikron) bis 50 µm (Mikron) hat.

5. Aerosolbehälter nach einem der vorhergehenden Ansprüche, wobei das Sonnenschutzmittel para-Aminobenzoesäure (PABA), Benzophenon-1, Benzophenon-2, Benzophenon-3, Benzophenon-4, Benzophenon-5, Benzophenon-6, Benzophenon-8, Benzophenon-9, Benzophenon-12, Ethyldihydroxypropyl-PABA, Glyceryl-PABA, Homosalat, Methylanthranilat, Octocrylen, Octyldimethyl-PABA, Octylmethoxycinnamat, Octylsalicylat, 2-Phenylbenzimidazol-5-sulfonsäure, Triethanolaminsalicylat, 4-Methylbenzylidencampher, rotes Petrolatum, Octylmethoxycinnamat, Ethylhexylmethoxycinnamat, Isoamyl-p-methoxycinnamat, Cinoxat, Dioxybenzon, Homosalat, Ethylhexylsalicylat, para-Aminobenzoesäure (PABA), Ethylhexyldimethyl-PABA, PEG-25-PABA, Benzylidencamphersulfonsäure, 3-Benzylidencampher, 4-Methylbenzylidencampher, Terephtalyliden-Dicamphersulfonsäure, Polyacylamidomethylbenzyliden, Phenylbenzimidazolsulfonsäure, 2-Hydroxy-4-methoxybenzophenon, Octocrylen, Menthylanthranilat, Ethylhexyltriazon, Diethylhexylbutamidotriazon, Butylmethoxydibenzoylmethan, Campherbenzalkoniummethosulfat, Methylen-bis-benzotriazolyl-tetramethylbutylphenol, Mononatriumsalz von 2,2-Bis-(1,4-Phenylen)-1H-benzimidazol-4, 6-Disulfonsäure, (1,3,5)-Triazin-2,4-bis-((4-(2-ethylhexyloxy)-2-hydroxy)-phenyl)-6-(4-methoxyphenyl), Polysilikon 15, DEA Methoxycinnamat, Digaloyltrioleat, Glyceryl-PABA, Isopropylbenzylsalicylat, Triethanolaminsalicylat, Glycerylethylhexanoat-di-methoxycinnamat, Methyldiisopropylcinnamat, Isopropylmethoxycinnamat, Diisopropylcinnamat-Estergemisch, Benzophenon-9, Trolaminsalicylat, Ethylenglycolsalicylat, Dipropylenglycolsalicylat, Methylsalicylat, Phenylsalicylat, Ethyl-PABA (Benzocain), Glyceryl-PABA, Amyldimethyl-PABA, Butylmethoxy-PABA, Phenylbenzimidazolsulfonsäure, Terephthalyliden-Dicamphersulfonsäure, Drometrizoltrisiloxansilatrizol, Campherbenzalkoniummethosulfat, Hososalat, Phenylbenzimidazolsulfonsäure, Terephthalyliden-Dicamphersulfonsäure, Butylmethoxydibenzoy-1-methan, Benzylidencamphersulfonsäure und -salze, Octorylen, Polyacrylamidmethylbenzylidencampher, Octylmethoxycinnamat, PEG-25-PABA, Isoamyl-p-methoxycinnamat, Octyltriazon, Drometrizoltrisiloxan, Dioctylbutamidotriazon, 4-Methylbenzylidencampher, 3-Benzylidencampher, Octylsalicylat, Octyldimethyl-PABA, Benzophenon-4, Benzophenon-5, Methylbis-benzotriazolyltetramethylbutylphenol, Bisymidazylat, Anisotriazin, Polysilikon-15 ist, oder Gemische von zwei oder mehreren davon.

## Revendications

1. Récipient d'aérosol capable de contenir un ou plusieurs gaz propulseurs, incluant une composition d'écran solaire et au moins un gaz propulseur, ledit gaz propulseur étant de l'éther diméthylique, ladite composition d'écran solaire offrant une protection contre les effets du soleil sur la peau d'une personne sur laquelle la composition est appliquée, dans lequel la composition d'écran solaire est une émulsion contenant au moins un agent de protection solaire, l'agent de protection solaire et le gaz propulseur étant en contact direct l'un avec l'autre dans l'émulsion et non séparés l'un de l'autre, le récipient étant pressurisé à une pression de 3,4 x 10⁴ Pa à 5,9 x 10⁵ Pa (de 20 à 100 psig), l'au moins un agent de protection solaire étant efficace pour protéger la peau contre les effets nuisibles du soleil lorsqu'il est en contact avec la peau d'une personne, la composition d'écran solaire pouvant être distribuée à partir du récipient sous la forme d'un spray ayant une taille de gouttelette de 0,01 µm (micron) à 5 000 µm (micron), la composition d'écran solaire étant efficace pour former un film sensiblement continu lorsqu'elle est appliquée sur la peau de la personne, dans lequel lorsque l'émulsion est distribuée à partir du récipient, l'agent de protection solaire forme des particules qui, au contact de la peau de la personne, forment un film sensiblement continu d'épaisseur uniforme, dans lequel l'émulsion est une émulsion d'huile dans l'eau ou d'eau dans l'huile dans l'eau.

2. Récipient d'aérosol selon la revendication 1, dans lequel la quantité d'agent de protection solaire dans l'émulsion est de 1 % à 90 %, de préférence de 20 % à 80 %, de manière préférée de 40 % à 70 %, et de manière préférée entre toutes de 60 % à 65 % du poids de la composition d'écran solaire.

3. Récipient d'aérosol selon l'une quelconque des revendications précédentes, dans lequel le récipient est à une pression située entre 1,0 x 10⁵ et 4,5 x 10⁵ Pa (entre 30 et 80 psig).

4. Récipient d'aérosol selon l'une quelconque des revendications précédentes dans lequel le spray a une taille de gouttelette de 1 µm (micron) à 50 µm (micron).

5. Récipient d'aérosol selon l'une quelconque des revendications précédentes dans lequel l'agent de protection solaire est l'acide para-aminobenzoïque (PABA), la benzophénone-1, la benzophénone-2, la benzophénone-3, la benzophénone-4, la benzophénone-5, la benzophénone-6, la benzophénone-8, la benzophénone-9, la benzophénone-12, l'éthyl dihydroxypropyl-PABA, le glycéryl PABA, l'homosalate, l'anthranilate de méthyle, l'octocrylène, l'octyl diméthyl PABA, le méthoxycinnamate d'octyle, le salicylate d'octyle, l'acide 2-phénylbenzimidazole-5-sulfonique, le salicylate de triéthanolamine, le 4-méthylbenzylidène camphre, le pétrolatum rouge, le méthoxycinnamate d'octyle, le méthoxycinnamate d'éthylhexyle, le p-méthoxycinnamate d'isoamyle, le cinoxate, la dioxybenzone, l'homosalate, le salicylate d'éthylhexyle, l'acide para-aminobenzoïque (PABA), l'éthylhexyl diméthyl PABA, le PEG-25 PABA, l'acide benzylidène camphre sulfonique, le 3-benzylidène camphre, le 4-méthyl benzylidène camphre, l'acide téréphtalylidène dicamphre sulfonique, le polyacylamidométhyl benzylidène, l'acide phénylbenzimidazole sulfonique, la 2-hydroxy-4-méthoxybenzophénone, l'octocrylène, l'anthranilate de menthyle, l'éthylhexyl triazone, la diéthylhexyl butamido triazone, le butyl méthoxydibenzoylméthane, le camphre benzalkonium méthosulfate, le méthylène bis-benzotriazolyl tétraméthyl butylphénol, un sel de monosodium de l'acide 2,2-bis-(1,4-phénylène)-1H-benzimidazole-4,6-disulfonique, le (1,3,5)-triazine-2,4-bis-((4-(2-éthylhexyloxy)-2-hydroxy)-phényl)-6-(4-méthoxyphényle), la polysilicone 15, le méthoxycinnamate de DEA, le trioléate de digaloyle, le glycéryl PABA, le salicylate d'isopropylbenzyle, le salicylate de triéthanolamine, l'éthylhexanoate diméthoxycinnamate de glycéryle, le cinnamate de méthyl diisopropyle, un mélange d'esters de méthoxy cinnamate d'isopropyle et de cinnamate de diisopropyle, la benzophénone-9, le salicylate de trolamine, le salicylate d'éthylène glycol, le salicylate de dipropylène glycol, le salicylate de méthyle, le salicylate de phényle, l'éthyl PABA (benzocaïne), le glycéryl PABA, l'amyl diméthyl PABA, le butyl méthoxy PABA, l'acide phénylbenzimidazole sulfonique, l'acide téréphtalylidène dicamphre sulfonique, le drométrizole trisiloxane silatrizole, le camphre benzalkonium méthosulpate, l'hososalate, l'acide phénylbenzimidazole sulfonique, l'acide téréphtalylidène dicamphre sulfonique, le butyle méthoxydibenzoyl méthane, l'acide benzylidène camphre sulfonique et ses sels, l'octorylène, le polyacrilamido-méthyl benzylidène camphre, le méthoxy-cinnamate d'octyle, le PEG-25-PABA, le p-méthoxy-cinnamate d'isoamyle, l'octyltriazone, le drométrizole trisiloxane, la dioctyl butamido triazone, le 4-méthylbenzylidène camphre, le 3-benzylidène camphre, le salicylate d'octyle, l'octyl diméthyl PABA, la benzophénone 4, la benzophénone 5, le méthyl bis-benzotriazolyl tétraméthyl butylphénol, le bisymidazylate, l'anisotriazine, la polysilicone-15, ou les mélanges de deux ou plusieurs de ceux-ci.
